# EUROPEAN PATENT SPECIFICATION

(11) **EP 1 356 828 B1**
(45) Date of publication and mention of the grant of the patent: **21.06.2006**
(21) Application number: 03008085.7
(22) Date of filing: 15.04.2003
(51) Int. Cl.: A61L 2/14

(54) **Sterilizing apparatus and method using the same**
Vorrichtung und Verfahren zum Sterilisieren
Dispositif et procédé de stérilisation

(30) Priority: 18.04.2002 JP 2002115912
(43) Date of publication of application: 29.10.2003
(73) Proprietor: TSURU GAKUEN Educational Institution, Hiroshima 731-5193 (JP)
(72) Inventor: Asahara, Yuji, Mitsubishi Heavy Ind., Ltd., Nishi-ku, Hiroshima, Hiroshima-ken (JP); Yoshida, Mitsuhiro, Mitsubishi Heavy Ind., Ltd., Nishi-ku, Hiroshima, Hiroshima-ken (JP); Katsura, Toshiaki, Mitsubishi Heavy Ind., Ltd., Nishi-ku, Hiroshima, Hiroshima-ken (JP); Wakamoto, Ikuo, Mitsubishi Heavy Ind., Ltd., Nishi-ku, Hiroshima, Hiroshima-ken (JP); Takagi, Toshinori, Hiroshima 731-5193 (JP); Tanaka, Takeshi, Hiroshima 731-5193 (JP)
(74) Representative: HOFFMANN EITLE

(56) References cited:
- WO-A-02/22180
- US-A- 3 600 126
- US-A- 6 162 405

## Description

### Background of the Invention

### 1. Field of the Invention

The present invention relates to a sterilizing apparatus and a sterilizing method using the same, and more particularly, to a sterilizing apparatus and a sterilizing method, in which a number of bacteria, bacterial endospores, yeast, viruses and molds attached to a substance can be decreased sharply in a short time.

### 2. Description of the Related Art

Various bacteria, bacterial endospores, yeast, viruses and molds adhere to a vessel. Appliances for which sterilization is needed are vessels such as a vessel for drops, a medicine bottle, and a pure water vessel, in which the bacteria should be not bred, medical treatment appliances such as a knife to which bacteria should not be adhered, and experiment appliances such as a flask in which bacteria should not be exist on the outer and inner surfaces. It is important to reduce bacteria on the surface of such an appliance, or viruses and molds adhered to a homely used article reliably and sharply.

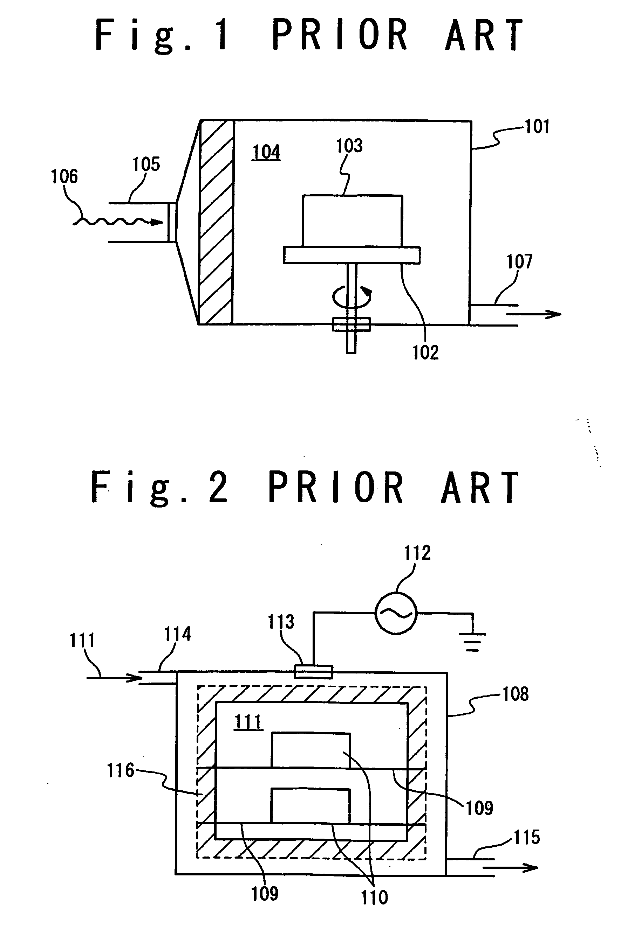

A sterilizing apparatus is known as a first conventional example for carrying out sterilization, as shown in Fig. 1. A target article 103 is located on a turntable 102 arranged in a vacuum chamber 101. A process gas 104 is introduced into the vacuum chamber 101, and micro wave 106 is applied through a wave guide 105. After the above process completes, the process gas 104 is exhausted through an exhaust pipe 107. The OH radicals as excited species in plasma generated in the vacuum chamber 101 are effective for sterilization of the bacteria adhering to the surface of the target article 103. The sterilization effect is a chemical effect. In addition, a sterilizing apparatus of a second conventional example shown in Fig. 2 is known.
Targets 110 are located on electrodes 109 arranged in a vacuum chamber 108, and a process gas 111 is introduced into the vacuum chamber 108. Moreover, power is supplied from a high frequency power supply 112 to the process gas in the vacuum chamber 108 through the electrode 113. The process gas 111 is introduced through an introduction pipe 114 and is exhausted through an exhaust pipe 115. The OH radicals as excited species in plasma 116 generated in the vacuum chamber 108 are effective the sterilization of the bacteria adhering to the surface of the process target 110.

Such a conventional sterilizing apparatus uses a high concentration hydrogen peroxide as the process gas 104 and 111 for the generation of the excited species having the high sterilization effect. To generate plasma and to sterilize the bacteria effectively, hydrogen peroxide has the concentration of 30% or higher. The hydrogen peroxide is thought as cancerogenic substance and needs to be processed in case of the high concentration. The OH radicals in the discharge plasma used in the conventional sterilizing apparatus has the sterilization effect through a chemical action. However, the sterilization efficiency by the chemical action is low because a radical quantity is less and the radicals diffuse.

Also, in the above-mentioned field in which remarkable decrease of the number of bacteria in an order of 4 or 6 digits or more is required, the time as much as 50 to 90 minutes is necessary for the sterilization. It is too long.

Also, in the conventional sterilizing apparatus, the shape of the plasma determined based on the electrode 102 or microwave mode is often nonconformity to the shape of the process target 103 or 110. Thus, the sterilization is local and un-uniform.

In addition, in the conventional sterilizing apparatus, a quantity of radicals, which come from the discharge region to the sterilization target, is not uniform, and the size of the process chamber is limited based on the lifetime of the radicals. Therefore, the realization of the process chamber with a large size is difficult, and the processing time becomes long.

Another apparatus for the sterilization of a target by using a plasma is known from US-A-6162405.

### Summary of the Invention

Therefore, an object of the present invention is to provide a sterilizing apparatus and a sterilizing method, in which the sterilization effect can be increased higher by using physical energy of particles in plasma in addition to a chemical effect with a high diffusion.

Another object of the present invention is to provide a sterilizing apparatus and a sterilizing method, in which a sterilization process can be carried out more uniformly.

Another object of the present invention is to provide a sterilizing apparatus and a sterilizing method, in which a sterilization effect can be made higher based on biological attributes of a cell or cell wall.

Another object of the present invention is to provide a sterilizing apparatus and a sterilizing method, in which the number of cells can be decreased reliably in an order of four digits or more.

In an aspect of the present invention, a sterilizing apparatus includes a chamber, first and second electrodes, a first AC power supply and a DC pulse power supply. The first electrode is provided in the chamber, and a target to be sterilized is supported by the first electrode. The first AC power supply is connected to the first electrode to supply AC power to the first electrode such that a plasma is generated around the first electrode. The DC pulse power supply is connected to the first electrode to supply DC pulse power to the first electrode such that ions or electrons are accelerated toward the target.

The sterilizing apparatus may further include a second electrode provided in the chamber to oppose to the first electrode, and a second AC power supply connected to the second electrode to supply AC power to the second electrode such that generation of the plasma is enhanced.

The sterilizing apparatus further includes an electrically conductive mesh provided to cover the target and connected to the first electrode.

Also, the first electrode may have an uneven surface, and the plasma is generated in a non-contact region between the first electrode and the target. Also, the first electrode may be a mesh electrode.

Also, the first electrode may have a shape covering an outer surface of the target.

Also, when the second electrode is provided in the chamber to oppose to the first electrode, the first electrode may be provided in a lower portion of the chamber and the second electrode may be provided in an upper portion of the chamber, and the target may be located on the first electrode. Oppositely, the first electrode may be provided in an upper portion of the chamber and the second electrode may be provided in a lower portion of the chamber. In this case, it is desirable that the first electrode has a support mechanism to hang the target.

Also, it is desirable that a process gas to be introduced into the chamber contains steam, and may further contain oxygen. In addition, the process gas may further contain hydrogen peroxide.

Also, the DC pulse power supply may generate a negative pulse to accelerate positive ions in the plasma toward the sterilized target, and generate a positive pulse to accelerate electrons and negative ions in the plasma toward the sterilized target, and both. In this case, one of a set of positive ions and a set of electrons and negative ions in the plasma is accelerated toward the sterilized target, and then the other is accelerated toward the sterilized target.

Also, the first AC power supply may apply the AC power to the first electrode in pulses to generate the plasma intermittently, and the first AC power supply may apply the AC power to the first electrode to generate the plasma continuously.

In another aspect of the present invention, a method of carrying out sterilization of a target, may be achieved by (a) supporting a target by a first electrode in a chamber; by (b) generating a plasma around the target between the first electrode and a second electrode in the chamber; and by (c) accelerating one of a set of positive ions and a set of electrons and negative ions in the plasma toward the sterilized target.

In this case, the step of (a) supporting may be achieved by supporting the target such that the target has a portion which does not contact the first electrode.

Also, the step of (b) generating may be achieved by supplying steam in the chamber as a process gas. In this case, the process gas further contains at least one of oxygen and hydrogen peroxide.

Also, the step of (b) generating may be achieved by generating the plasma intermittently and periodically. Alternately, the step of (b) generating may be achieved by generating the plasma continuously.

Also, the step of (c) accelerating may be achieved by applying a negative pulse to the first electrode such that the set of positive ions are accelerated toward the target. Also, the step of (c) accelerating may be achieved by applying a positive pulse to the first electrode such that the set of negative ions and electrodes are accelerated toward the target.

### Brief Description of the Drawings

Fig. 1 is a front cross sectional view showing a sterilizing apparatus of a first conventional example;
Fig. 2 is a front cross sectional view showing a sterilizing apparatus of a second conventional example;
Fig. 3 is a front cross sectional view showing a sterilizing apparatus according to a first embodiment having some features of the present invention;
Fig. 4A is a diagram showing a waveform of AC power applied, and Figs. 4B to 4D are diagrams showing waveforms showing DC power applied;
Fig. 5 is a front cross sectional view showing plasma generated in a chamber;
Fig. 6 is a front cross sectional view showing the sterilizing apparatus according to a second embodiment having some features of the present invention;
Fig. 7 is a front cross sectional view showing the sterilizing apparatus according to having some features an embodiment of the present invention;
Fig. 8 is a front cross sectional view showing the sterilizing apparatus according to a fourth embodiment having some features of the present invention;
Fig. 9 is a front cross sectional view showing the sterilizing apparatus according to a fifth embodiment having some features of the present invention; and
Fig. 10 is a front cross sectional view showing the sterilizing apparatus according to a sixth embodiment having some features of the present invention.

### Description of the Preferred Embodiments

Hereinafter, the sterilizing apparatus according to the present invention will be described in detail with reference to the attached drawings.

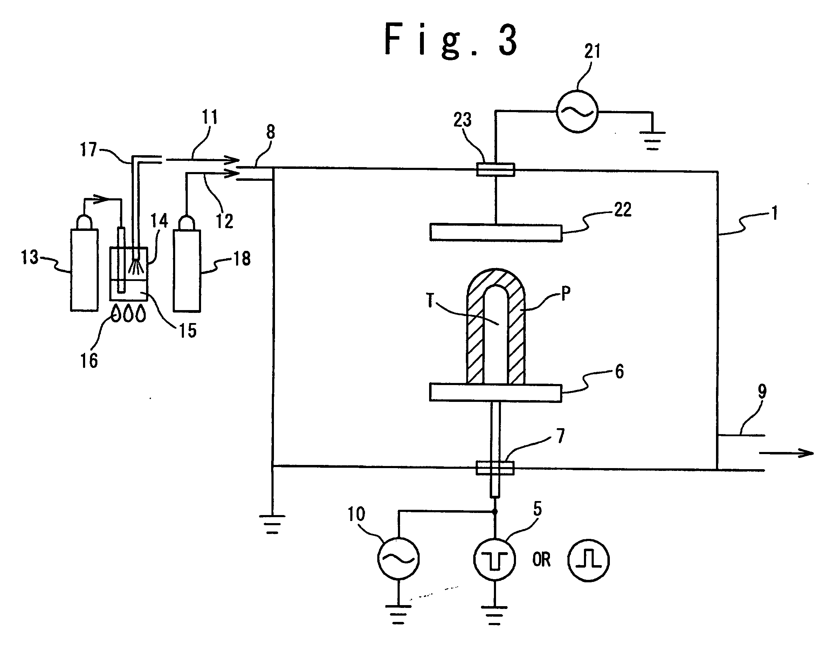

Fig. 3 shows the structure of the sterilizing apparatus according to a first embodiment having some features of the present invention. The sterilizing apparatus in the first embodiment is composed of a first electrode 6 and a second electrode 22 provided in a chamber 1. The vacuum chamber 1 is grounded. A target T to be sterilized is located on the first electrode 6. The sterilizing apparatus is composed of a DC power supply 5 and a high frequency AC power supply 10, which are provided between the first electrode 6 and the ground. The sterilizing apparatus is desirably further composed of a high frequency AC power supply 21 provided between the second electrode 22 and the ground. A process gas is introduced into the chamber 1 through a gas introduction port 8 and is exhausted from the chamber 1 through a gas exhaust port 9.

In the sterilizing apparatus described above, the vacuum chamber 1, the AC power supply 10 and the DC power supply 5 are used for plasma sterilization. The AC power supply 10 applies AC power to the first electrode 6 through an introduction terminal 7 attached to the wall of the vacuum chamber 1 to generate a plasma. The generated plasma has a diffusion property and a quantity of radicals as excitation species of the plasma is increased. Therefore, the plasma has the same chemical sterilization function as in the conventional examples. Also, the AC power supply 21 applies AC power to the second electrode 22 through an introduction terminal 23 attached to the wall of the vacuum chamber 1 to generate plasma in the whole of the vacuum chamber 1 or in a wide region. The plasma generated from the second electrode 22 has the same chemical sterilization function as in the conventional examples, too. The DC power supply 5 restrains the diffusion of the plasma.

The target T to be sterilized is located on the first electrode 6, as described above. When the target T is electrically conductive, the target T functions as a part of the first electrode 6. The vacuum chamber 1 is grounded.

The gas introduction port 8 and the gas exhaust port 9 are provided for the vacuum chamber 1. It is desirable that a filter (not shown) is provided for each of the gas introduction port 8 and the gas exhaust port 9 to prevent dust particles with germs from entering the vacuum chamber 1. The process gas is introduced from the gas introduction port 8, and the process gas is composed of a first process gas 11 and a second process gas 12. It is desirable that the process gas is a mixture gas of steam, oxygen gas and nitrogen gas. They may be used independently and air may be used. Air is enclosed into a first cylinder 13, and is emitted into water 15 in a steam generator 14 through a valve and a pressure reducing valve (both not shown). The water 15 is heated by a heater 16 in the steam generator 14, and the steam is introduced into the vacuum chamber 1 through a supply pipe 17 as the first process gas 11 together with the oxygen gas and nitrogen gas. The second process gas 12 is enclosed into a second cylinder 18. A dilute gas is desirably used as the second process gas 12.

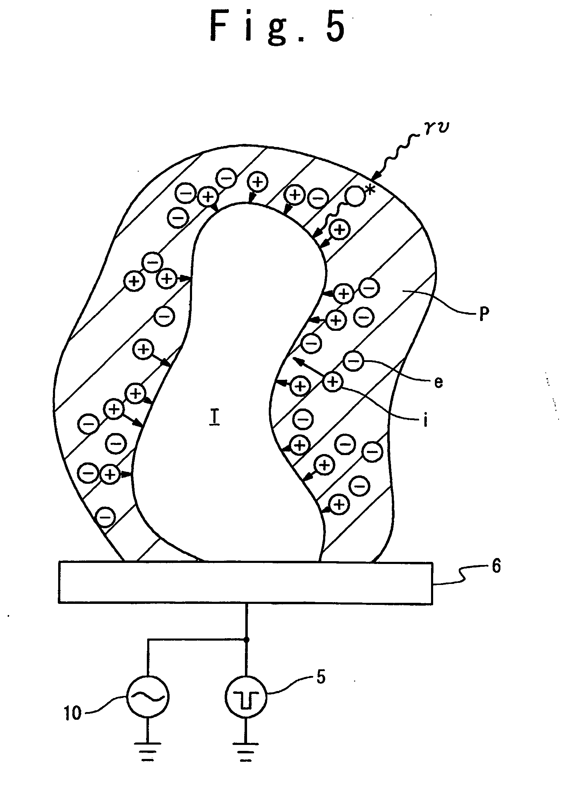

The steam, oxygen, nitrogen and dilute gases are introduced into the vacuum chamber 1, and the DC pulse power and the AC power are applied from the DC power supply 5 and the AC power supply 10 to the first electrode 6 as described above. As a result, as shown in Fig. 5, when the target T is electrically conductive, the plasma P is uniformly generated in the neighborhood region of the target T, by the proper setting of the application condition of the powers and the process gas pressure while keeping the high voltage without un-uniform discharge and arc discharge.

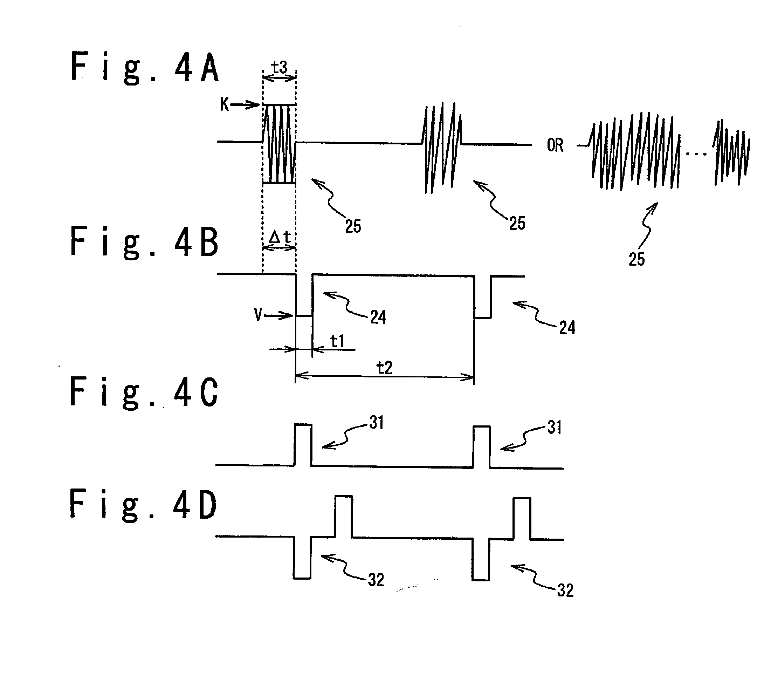

Figs. 4A and 4B show the desirable power waveforms of the AC pulse power supplied from the AC power supply 10 and the DC pulse power supplied from the DC power supply 5. As shown in Fig. 4A, the AC power 25 supplied from the AC power supply 10 may be an AC pulse power or a continuous AC power. Also, as shown in Fig. 4B, the DC power supply 5 generates the DC pulse power 24 at a period t2. The DC pulse power has a DC negative voltage V and the pulse duration (width) t1. It is desirable that the width of the DC pulse 24 is the order of a few to a few tens of µs, and the maximum voltage is about tens of kV. However, the peak value of the electric current is adjusted to be below set values of circuit components. It is desirable that the repetitive period t2 of the DC pulse 24 is as much as hundreds pps to thousands of pps.

As described above, the AC power supply 10 generates the AC power 25 periodically in a pulse or continuously. Thus, the plasma is generated periodically intermittently or continuously. As the RF condition of the AC pulse power 25, the frequency is set to f, the peak voltage is set to K, and the pulse duration is set to t3. The DC pulse power 24 rises up with the delay time Δt after the AC pulse power 25 rises up. By adjusting parameters of the negative voltage pulse condition of the DC pulse power 24 and RF condition of the AC pulse power 25, an ion injection energy distribution, an energy peak, and a plasma density are adjusted. Through the adjustment, the sterilization time until a ruled sterilization percentage is achieved can be controlled. By adjusting the duty ratio of the DC pulse power 24, i.e., the period t2, an ion flux incident to the target T in a unit time can be more effectively controlled in the sterilization process, and the sterilization speed can be controlled.

The AC pulse power 25 is applied to the first electrode 6 temporally preceding to application of the DC pulse power 24. The plasma is generated around the first electrode 6 and the target T as the conductor electrically coupled to the first electrode 6. The DC pulse power 24 is applied to the first electrode 6 temporally after the application of the AC pulse power 25 by the time Δt. The positive ions and electrons of the plasma P around the target T receive electrostatic force. The electrons are repelled forcedly from the surface of the target T or the peripheral region of the target T to go away from the target T. The positive ions are attracted to the target T and accelerated toward the target T by the plasma sheath existing around the target T. The accelerated positive ions inflict damage on the bacteria cell existing on the surface of the target T. The damage is caused by driving of the ions having the kinetic energy as physical energy into the cell wall or the cell. When the first electrode 6 is charged positively, electrons are driven into the cell wall or the cell. In this way, the bacteria perish with the physical effect.

The DC pulse 24 is used to generate plasma in the neighborhood region of the target T through the self-discharge. The AC pulse power 25 increases the excitation energy in the plasma generated based on the DC pulse power 24, and increases a quantity of the excitation species and radicals supplementarily and in a wide region. The plasma sheath formed by the self-discharge of the DC pulse power 24 which is applied to the first electrode 6 under the existence of the plasma has the shape corresponding to the surface shape of the target T. The plasma sheath forms an electrically accelerating field to uniformly drive the positive ions or electrons toward the surface of the target T with various different shapes such as an uneven surface shape. The uniformity of the electrically accelerating field gives the uniform sterilization ability to the whole surface of the target T. The addition of the second electrode 22 and the AC power supply 21 increases the excitation species in the plasma generated in a wide region. The radicals diffuses into the wide region and are driven toward the target T, resulting in the improvement of the efficiency. Thus, the process time until a preset sterilization percentage is achieved can be reduced.

The plasma sheath is lost during the dwelling time of the DC pulse power 24 in the period t2. At this time, radicals as the excitation species activated in the plasma P due to the after-glow exist in the neighborhood of the target T and reach the surface of the target T. Thus, the bacteria perish in a high efficiency. This effect is based on the chemical effect that is the same effect as in the conventional examples. In this way, according to the present invention, the number of bacteria can be decreased in the order of six digits through the multiple effects of the physical effect and the chemical effect.

Such radicals are the OH radicals obtained from steam or hydrogen peroxide steam, the oxygen radicals obtained from the oxygen gas and ozone. By using water and hydrogen peroxide as source substances of the radical generation, the reliable sterilization effect can be expected. Therefore, the process gas may contain hydrogen peroxide. When it is possible to process using water and harmless gas, the chemical process can be made safe remarkably. Moreover, the chemical sterilization effect can be reliably achieved using the OH radicals generated through dissolution of a mixture gas of steam or a little quantity of gas of hydrogen peroxide without strongly depending on harmful hydrogen peroxide.

In the above description, almost the same sterilization effect can be achieved even if the positive DC pulse power 25 is used in place of the negative DC pulse power 24, as shown in Fig. 4C. The parameters are determined in consideration of an electric charge movement speed on the surface of the target T (in case of an insulator) which has an influence on the pulse duration t1 of the DC pulse power 25, and an electric charge extinguishment speed which is caused by the interaction between the target T and particles in the plasma.

Also, the DC pulse power 32 with a positive pulse and a negative pulse may be used in place of the negative DC pulse power 24 and the positive DC pulse power 31, as shown in Fig. 4D. In this case, in addition to the positive ions, negative ions and electrons can be used for sterilization. Therefore, the higher sterilization effect can be achieved.

Also, a light source (not shown) may be used in place of the AC power supply 21. It is desirable that the light source emits light with various wavelengths from the infrared to the ultraviolet into the inside of the chamber 1. In this case, the second electrode 22 is connected to the ground.

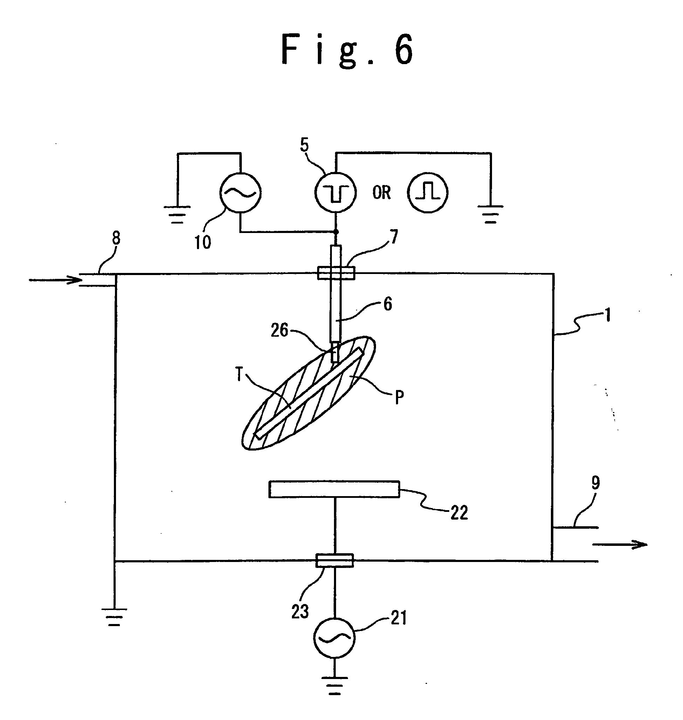

Fig. 6 shows the sterilizing apparatus according to a second embodiment having some features of the present invention. The first electrode 6 is provided in the upper portion of the chamber 1 and the second electrode 22 is provided in the lower portion of the chamber 1. The AC power supply 10 and the DC power supply 5 are connected to the first electrode 6 and the AC power supply 21 is connected to the second electrode 22. The first electrode 6 in the second embodiment is replaced to a suspending type electrode from a plate type electrode in the first embodiment. A conductive suspending mechanism 26 is added to the first electrode 6. The suspending mechanism 26 may be replaced by a mechanism of a sandwiching type or an absorbing type. The target T is suspended, or sandwiched or absorbed by the suspending mechanism 26. The suspending, sandwiching or absorbed position is changed depending on the target T.

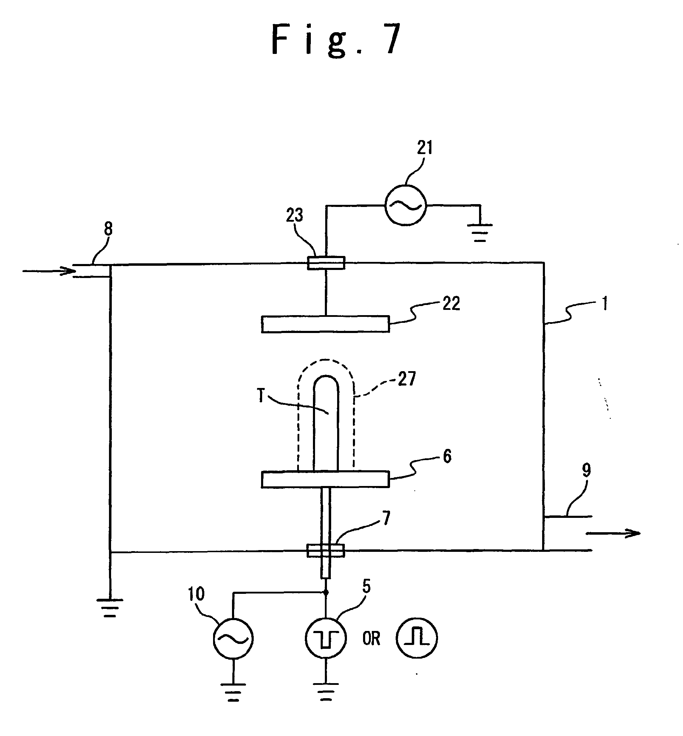

Fig. 7 shows the sterilizing apparatus according to an embodiment of the present invention. The sterilizing apparatus in this embodiment can be suitably applied in case the target T is non-conductor or dielectric. This embodiment is similar to the first embodiment. In this embodiment, a mesh electrode 27 is located on the first electrode 6 to cover the target T, such that the mesh electrode 27 is connected with the first electrode 6. The DC pulse power 24 and the AC power 5 25 are supplied to the mesh electrode 27 in the above-mentioned time difference Δt, or simultaneously after the above-mentioned time difference. Thus, the electrons or positive or negative ions are accelerated toward the target T and driven into the target T through the mesh electrode 27.

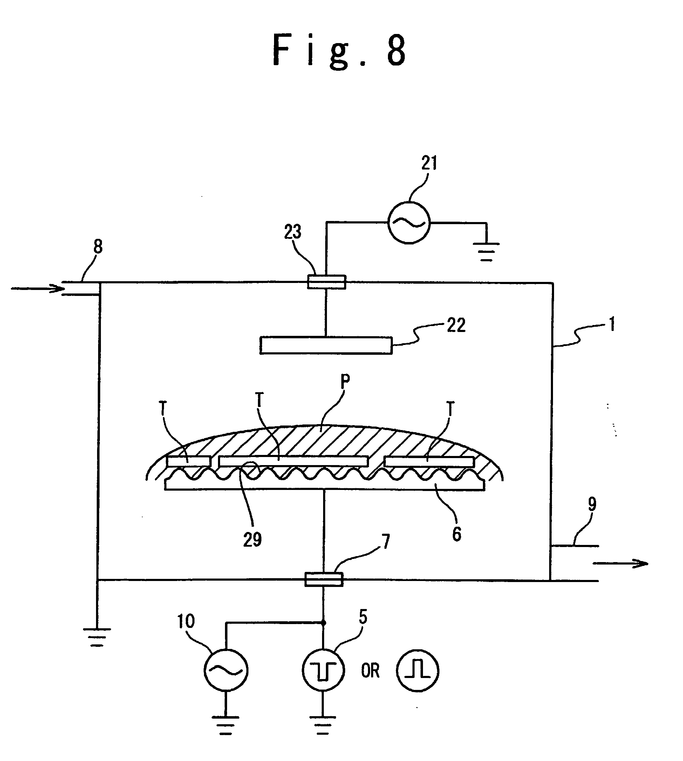

Fig. 8 shows the sterilizing apparatus according to a fourth embodiment having some features of the present invention. The fourth embodiment is the same as the first embodiment in the electrical connection and arrangement of the electrodes. In the fourth embodiment, a plurality of targets T are located on the first electrode 6. The upper surface plane of the first electrode 6 is formed to have a waveform shape or lattice shape such that the plasma P goes around to the side and back of the target T. The target T is supported at numerous points on the first electrode 6.

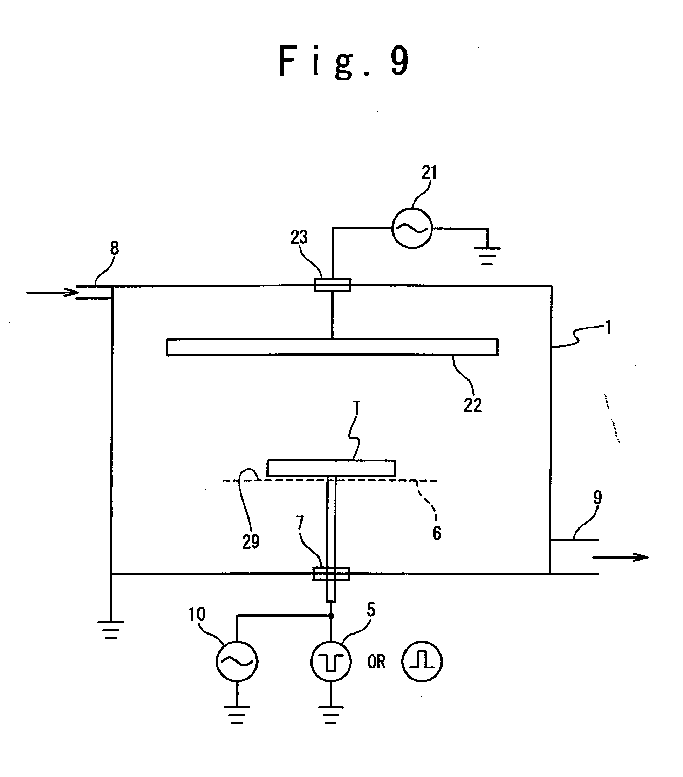

Fig. 9 shows the sterilizing apparatus according to a fifth embodiment having some features of the present invention. The fifth embodiment is the same as the first embodiment in the electrical connection and position of the electrodes. However, in the fifth embodiment, a mesh electrode is used as the first electrode in place of the plate electrode. The surface of the first electrode 6 on which the target T is supported is desirably formed to have an uneven surface or a lattice surface.

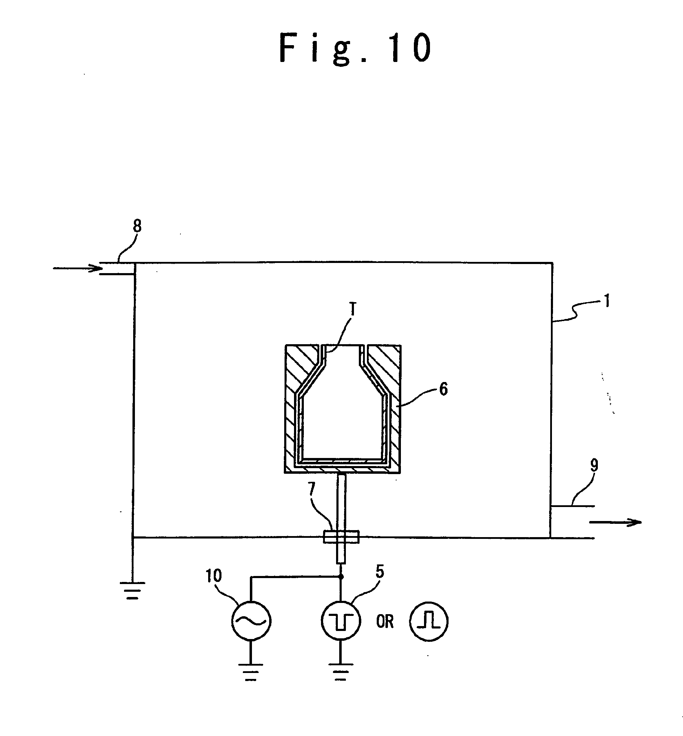

Fig. 10 shows the sterilizing apparatus according to a sixth embodiment having some features of the present invention. A non-conductive drinking water vessel is exemplified as the target T. The first electrode 6 has the inner shape to match to the outer surface of the target T. If the target T is dielectric, discharge is formed inside the vessel by the electric field generated inside the target T by the AC power supply 10 and the DC power supply 5. When the vessel is a non-conductor and processed in the outer surface, the first electrode 6 is replaced with a mesh electrode. If the vessel is a conductor, the AC power and the DC pulse power are applied to the vessel itself.

Although the embodiments of the present invention are described above, the present invention is not limited to them. Various modifications would be possible to the person skilled in the art .

For example, by arranging a plurality of first electrodes in the vacuum chamber 1 and forming a plurality of plasma sheaths, a plurality of process objects can be efficiently processed while using the excitation energy of the plasma generated in a wide region.

The sterilizing apparatus and the method of sterilizing of the present invention has the improvement of the sterilization effect by destroying the cells of the bacteria chemically and physically. Especially, the sterilization effect in the order of four digits or more is achieved.

## Claims

1. A sterilizing apparatus comprising:
a chamber (1);
a first electrode (6) provided in said chamber, a target to be sterilized being supported by said first electrode;
a first AC power supply (10) connected to said first electrode to supply AC power to said first electrode such that a plasma is generated around said first electrode;
a DC pulse power supply (5) connected to said first electrode to supply DC pulse power to said first electrode such that ions or electrons are accelerated toward said target; and
an electrically conductive mesh (27) provided to cover said target and connected to said first electrode.

2. The sterilizing apparatus according to claim 1, further comprising:
a second electrode (22) provided in said chamber to oppose to said first electrode; and
a second AC power supply (21) connected to said second electrode to supply AC power to said second electrode such that generation of said plasma is enhanced.

3. The sterilizing apparatus according to any of claims 1 and 2, wherein said first electrode has an uneven surface (29), and said plasma is generated in a non-contact region between said first electrode and said target.

4. The sterilizing apparatus according to any of claims 1 to 3, wherein said first electrode is a mesh electrode.

5. The sterilizing apparatus according to any of claims 1 to 3, wherein said first electrode has a shape covering an outer surface of said target.

6. The sterilizing apparatus according to any of claims 1 to 5, further comprising:
a second electrode provided in said chamber to oppose to said first electrode, and
wherein said first electrode is provided in a lower portion of said chamber and said second electrode is provided in an upper portion of said chamber, and said target is located on said first electrode.

7. The sterilizing apparatus according to any of claims 1 to 5, further comprising:
a second electrode provided in said chamber to oppose to said first electrode, and
wherein said first electrode is provided in an upper portion of said chamber and said second electrode is provided in a lower portion of said chamber, and
said first electrode has a support mechanism (26) to hang said target.

8. A method of carrying out sterilization of a target in an apparatus according to any one of the preceding claims, comprising the steps of:
(a) supporting a target by a first electrode in a chamber;
(b) generating a plasma around said target between said first electrode and a second electrode in said chamber; and
(c) accelerating one of a set of positive ions and a set of electrons and negative ions in said plasma toward said sterilized target.

9. The method according to claim 8, wherein said step of (a) supporting comprises the step of:
supporting said target such that said target has a portion which does not contact said first electrode.

10. The method according to claim 8 or 9, wherein said step of (b) generating comprises the step of:
supplying steam in said chamber as a process gas.

11. The method according to claim 10, wherein said process gas further contains at least one of oxygen and hydrogen peroxide.

12. The method according to any of claims 8 to 11, wherein said step of (b) generating comprises the step of:
generating said plasma intermittently.

13. The method according to any of claims 8 to 11, wherein said step of (b) generating comprises the step of:
generating said plasma intermittently and periodically.

14. The method according to any of claims 8 to 11, wherein said step of (b) generating comprises the step of:
generating said plasma continuously.

15. The method according to any of claims 8 to 14, wherein said step of (c) accelerating comprises the step of:
applying a negative pulse to said first electrode such that the set of positive ions are accelerated toward said target.

16. The method according to any of claims 8 to 14, wherein said step of (c) accelerating comprises the step of:
applying a positive pulse to said first electrode such that the set of negative ions and electrodes are accelerated toward said target.

17. The sterilizing apparatus according to any of claims 8 to 16, wherein said DC pulse power supply generates a positive pulse and a negative pulse such that one of a set of positive ions and a set of electrons and negative ions in said plasma is accelerated toward said sterilized target, and then the other is accelerated toward said sterilized target.

## Patentansprüche

1. Sterilisationsvorrichtung, umfassend:
eine Kammer (1);
eine erste in der Kammer vorgesehene Elektrode (6), ein von der ersten Elektrode abgestütztes zu sterilisierendes Ziel;
eine erste Wechselstrom-Energiequelle (10), die mit der ersten Elektrode verbunden ist, um Wechselstrom-Energie auf die erste Elektrode so zuzuführen, dass ein Plasma um die erste Elektrode herum erzeugt wird;
eine Gleichstrom-Pulsenergiequelle (5), die mit der ersten Elektrode verbunden ist, um eine Gleichstrom-Pulsenergie auf die erste Elektrode so zuzuführen, dass Ionen oder Elektronen auf das Ziel hin beschleunigt werden; und
ein elektrisch leitfähiges Gitter (27), das dazu vorgesehen ist das Ziel abzudecken und das mit der ersten Elektrode verbunden ist.

2. Sterilisationsvorrichtung gemäß Anspruch 1, des Weiteren umfassend:
eine zweite Elektrode (22), die in der Kammer vorgesehen ist, um der ersten Elektrode gegenüberzustehen; und
eine zweite Wechselstrom-Energiequelle (21), die mit der zweiten Elektrode verbunden ist, um eine Wechselstrom-Energie auf die zweite Elektrode zuzuführen, so dass die Erzeugung des Plasmas gefördert wird.

3. Sterilisationsvorrichtung gemäß einem der Ansprüche 1 und 2, wobei die erste Elektrode eine unebene Oberfläche (29) aufweist und das Plasma in einem Nicht-Kontakt-Bereich zwischen der ersten Elektrode und dem Ziel erzeugt wird.

4. Sterilisationsvorrichtung gemäß einem der Ansprüche 1 bis 3, wobei die erste Elektrode eine Gitterelektrode ist.

5. Sterilisationsvorrichtung gemäß einem der Ansprüche 1 bis 3, wobei die erste Elektrode eine Form aufweist, die eine äußere Oberfläche des Ziels abdeckt.

6. Sterilisationsvorrichtung gemäß einem der Ansprüche 1 bis 5, des Weiteren umfassend:
eine zweite Elektrode, die in der Kammer so vorgesehen ist, dass sie der ersten Elektrode gegenübersteht, und
wobei die erste Elektrode in einem unteren Abschnitt der Kammer vorgesehen ist und die zweite Elektrode in einem oberen Abschnitt der Kammer vorgesehen ist und das Ziel auf der ersten Elektrode platziert ist.

7. Sterilisationsvorrichtung gemäß einem der Ansprüche 1 bis 5, des Weiteren umfassend:
eine zweite Elektrode, die in der Kammer so vorgesehen ist, dass sie der ersten Elektrode gegenübersteht, und
wobei die erste Elektrode in einem oberen Abschnitt der Kammer vorgesehen ist und die zweite Elektrode in einem unteren Abschnitt der Kammer vorgesehen ist, und
die erste Elektrode einen Abstützmechanismus (26) aufweist, um das Ziel aufzuhängen.

8. Verfahren zur Durchführung der Sterilisation eines Ziels in einer Vorrichtung gemäß einem der voranstehenden Ansprüche, umfassend die Schritte:
(a) Abstützen eines Ziels durch eine erste Elektrode in einer Kammer;
(b) Erzeugen eines Plasmas zwischen der ersten Elektrode und einer zweiten Elektrode in der Kammer um das Ziel herum; und
(c) Beschleunigen eines Satzes positiver Ionen und eines Satzes von Elektronen und negativen Ionen in dem Plasma auf das sterilisierte Ziel hin.

9. Verfahren gemäß Anspruch 8, wobei der Schritt (a) des Abstützens den folgenden Schritt umfasst:
Abstützen des Ziels, so dass das Ziel einen Abschnitt aufweist, der nicht die erste Elektrode berührt.

10. Verfahren gemäß Anspruch 8 oder 9, wobei der Schritt (b) des Erzeugens den folgenden Schritt umfasst:
Zuführen von Dampf in die Kammer als Prozessgas.

11. Verfahren gemäß Anspruch 10, wobei das Prozessgas des Weiteren zumindest entweder Sauerstoff und/oder Wasserstoffperoxyd enthält.

12. Verfahren gemäß einem der Ansprüche 8 bis 11, wobei der Schritt (b) des Erzeugens den folgenden Schritt umfasst:
Erzeugen des Plasmas mit Unterbrechungen.

13. Verfahren gemäß einem der Ansprüche 8 bis 11, wobei der Schritt (b) des Erzeugens den folgenden Schritt umfasst:
Erzeugen des Plasmas mit Unterbrechungen und periodisch.

14. Verfahren gemäß einem der Ansprüche 8 bis 11, wobei der Schritt (b) des Erzeugens den folgenden Schritt umfasst:
kontinuierliches Erzeugen des Plasmas.

15. Verfahren gemäß einem der Ansprüche 8 bis 14, wobei der Schritt (c) des Beschleunigen den folgenden Schritt umfasst:
Aufbringen eines negativen Pulses auf die erste Elektrode, so dass der Satz positiver Ionen auf das Ziel hin beschleunigt wird.

16. Verfahren gemäß einem der Ansprüche 8 bis 14, wobei der Schritt (c) des Beschleunigens den folgenden Schritt umfasst:
Aufbringen eines positiven Pulses auf die erste Elektrode, so dass der Satz negativer Ionen und die Elektronen auf das Ziel hin beschleunigt werden.

17. Sterilisationsvorrichtung gemäß einem der Ansprüche 8 bis 16, wobei die Gleichstrom-Pulsenergiequelle einen positiven Puls sowie einen negativen Puls erzeugt, so dass ein Ion eines Satzes positiver Ionen und ein Elektron oder Ion eines Satzes von Elektronen und negativen Ionen in dem Plasma auf das sterilisierte Ziel hin beschleunigt wird und dann das andere auf das sterilisierte Ziel hin beschleunigt wird.

## Revendications

1. Appareil de stérilisation comprenant:
une chambre (1);
une première électrode (6) fournie dans ladite chambre, une cible à stériliser étant supportée au moyen de ladite électrode;
une première alimentation en courant alternatif (10) connectée à ladite première électrode pur fournir une alimentation en alternatif à ladite première électrode de telle façon qu'un plasma soit généré autour de ladite première électrode;
une alimentation en impulsion continue (5) connectée à ladite première électrode pour fournir une alimentation en impulsion continue à ladite première électrode de telle façon que les ions ou les électrons soient accélérés vers ladite cible; et
un maillage électriquement conducteur (27) fourni pour couvrir ladite cible et connecté à ladite première électrode.

2. Appareil de stérilisation selon la revendication 1, comprenant de plus:
une deuxième électrode (22) fournie dans ladite chambre pour s'opposer à ladite première électrode ; et
une deuxième alimentation en courant alternatif (21) connectée à ladite deuxième électrode pour fournir l'alimentation en alternatif à ladite deuxième électrode de telle façon que la génération dudit plasma est améliorée.

3. Appareil de stérilisation selon l'une quelconque des revendications 1 et 2, dans lequel ladite première électrode a une surface inégale (29), et ledit plasma est généré dans une région dépourvue de contact entre ladite première électrode et ladite cible.

4. Appareil de stérilisation selon l'une quelconque des revendications 1 à 3, dans lequel ladite première électrode est une grille à mailles.

5. Appareil de stérilisation selon l'une quelconque des revendications 1 et 3, dans lequel ladite première électrode a une forme couvrant une surface extérieure de ladite cible.

6. Appareil de stérilisation selon l'une quelconque des revendications 1 et 5, comprenant de plus:
une deuxième électrode fournie dans ladite chambre pour s'opposer à ladite première électrode, et
dans lequel ladite première électrode est fournie dans une partie inférieure de ladite chambre et ladite deuxième électrode est fournie dans une partie supérieure de ladite chambre, et ladite cible est placée sur ladite première électrode.

7. Appareil de stérilisation selon l'une quelconque des revendications 1 et 5, comprenant de plus :
une deuxième électrode fournie dans ladite chambre pour s'opposer à ladite première électrode, et
dans lequel ladite première électrode est fournie dans une partie supérieure de ladite chambre et ladite deuxième électrode est fournie dans une partie inférieure de ladite chambre, et
ladite première électrode a un mécanisme de support (26) pour suspendre ladite cible.

8. Procédé de réalisation de la stérilisation d'une cible dans un appareil selon l'une quelconque des revendications précédentes, comprenant les étapes de:
(a) support d'une cible au moyen d'une première électrode dans une chambre;
(b) génération d'un plasma autour de ladite cible entre ladite première électrode et une deuxième électrode dans ladite chambre; et
(c) accélération d'un parmi un ensemble d'ions positifs et d'un ensemble d'électrons et d'ions négatifs dans ledit plasma vers ladite cible stérilisée.

9. Procédé selon la revendication 8, dans lequel ladite étape (a) de support comprend l'étape de :
support de ladite cible de telle façon que ladite cible ait une partie qui ne soit pas en contact avec ladite première électrode.

10. Procédé selon la revendication 8 ou 9, dans lequel ladite étape de (b) génération comprend l'étape de:
fourniture de vapeur dans ladite chambre comme gaz de traitement.

11. Procédé selon la revendication 10, dans lequel ledit gaz de traitement comprend de lus au moins un parmi l'oxygène et le peroxyde d'hydrogène.

12. Procédé selon l'une quelconque des revendications 8 à 11, dans lequel ladite étape de (b) génération comprend l'étape de:
génération dudit plasma de façon intermittente.

13. Procédé selon l'une quelconque des revendications 8 à 11, dans lequel ladite étape de (b) génération comprend l'étape de:
génération dudit plasma de façon intermittente et de façon périodique.

14. Procédé selon l'une quelconque des revendications 8 à 11, dans lequel ladite étape de (b) génération comprend l'étape de :
génération dudit plasma de façon continue.

15. Procédé selon l'une quelconque des revendications 8 à 14, dans lequel ladite étape (c) d'accélération comprend l'étape de :
application d'une impulsion négative à ladite première électrode de telle façon que l'ensemble des ions positifs soit accéléré vers ladite cible.

16. Procédé selon l'une quelconque des revendications 8 à 14, dans lequel ladite étape (c) d'accélération comprend l'étape de :
application d'une impulsion positive à ladite première électrode de telle façon que l'ensemble des ions négatifs et des électrodes soient accélérés vers ladite cible.

17. Appareil de stérilisation selon l'une quelconque des revendications 8 à 16, dans lequel ladite alimentation en impulsion continue génère une impulsion positive et une impulsion négative de telle façon que l'un parmi un ensemble d'ions positifs et d'un ensemble d'électrons et d'ions négatifs dans ledit plasma soit accéléré vers ladite cible stérilisée, puis que l'autre soit accéléré vers ladite cible stérilisée.
